# EUROPEAN PATENT SPECIFICATION

(11) **EP 1 535 895 B1**
(45) Date of publication and mention of the grant of the patent: **25.04.2012**
(21) Application number: 03741207.9
(22) Date of filing: 04.07.2003
(51) Int. Cl.: C07C 67/313, C07C 69/747

(54) **PROCESS FOR PRODUCTION OF FORMYLCYCLOPROPANECARBOXYLIC ESTERS**
VERFAHREN ZUR HERSTELLUNG VON FORMYLCYCLOPROPANCARBONSÄUREESTERN
PROCEDE DE PRODUCTION D'ESTERS FORMYLCYCLOPROPANECARBOXYLIQUES

(30) Priority: 17.07.2002 JP 2002208056
(43) Date of publication of application: 01.06.2005
(73) Proprietor: Sumitomo Chemical Company, Limited, Tokyo 104-8260 (JP)
(72) Inventor: MINAMIDA, Ryo, Kyotanabe-shi, Kyoto 610-0357 (JP); HAGIYA, Koji, Ibaraki-shi, Osaka 567-0833 (JP); ITAGAKI, Makoto, Katano-shi, Osaka 576-0066 (JP)
(74) Representative: Henkel, Breuer & Partner
(86) International application number: PCT/JP2003/008555
(87) International publication number: WO 2004/007419

(56) References cited:
- JP-A- 5 310 632
- JP-A- 7 330 654
- JP-A- 9 087 226
- JP-A- 10 237 006
- US-A- 5 821 374
- OGIBIN, YU. N. ET AL: "Electrochemical and chemical oxidation of alcohols in a two-phase system using N-oxopiperidinium salt: synthesis of 4-chlorobutanal, formylcyclopropanes, and m-phenoxybenzaldehyde" BULLETIN OF THE RUSSIAN ACADEMY OF SCIENCES. DIVISION OF CHEMICAL SCIENCE., vol. 41, no. 4, 1992, pages 735-739, XP008065147 USPLENUM PUBLISHING CO, NEW YORK, NY.
- SEMMELHACK, M. F. ET AL: "Nitroxyl -mediated electrooxidation of alcohols to aldehydes and ketones" JOURNAL OF THE AMERICAN CHEMICAL SOCIETY , 105(13), 4492-4 CODEN: JACSAT; ISSN: 0002-7863, 1983, XP002385263
- OSTERGAARD N. ET AL.: 'Scope and limitations of chiral bis(oxazoline) ligands in the copper-catalysed asymmetric cyclopropanation of trisubstituted alkens' TETRAHEDRON vol. 57, 2001, pages 6083 - 6088, XP004251451

## Description

### Technical Field

The present invention relates to a process for production of a formylcyclopropanecarboxylate compound.

### Background Art

As a production method of methyl or ethyl 2,2-dimethyl-3-formylcyclopropanecarboxylate, there have been known a method of oxidizing methyl or ethyl 2,2-dimethyl-3-(hydroxymethyl)cyclopropanecarboxylate with pyridinium chlorochromate (Heterocycles, 23, 2859 (1985)), and a Swern oxidation reaction(Tetrahedron, 57, 6083 (2001)), etc. Since the oxidation with pyridinium chlorochromate has problems of waste-treatment due to the use of a heavy metal-containing reagent, and Swern oxidation has problems in that the control of the temperature was difficult due to large heat of the reaction and byproduction of the dimethyl sulfide which is a malodorous substance either of them have not always been satisfactory.

N. OSTERGAARD et al., "Scope and limitations of chiral bis(oxazoline) ligands in the copper-catalysed asymmetric cyclopropanation of trisubstituted alkenes", Tetrahedron, 2001, Vol. 57, pages 6083 to 6088 report that a series of derivatives of 3-methyl-2-buten-1-ol has been used to test the scope and limitations of the copper-catalysed asymmetric cyclopropanation of trisubstituted alkenes by ethyl diazoacetate in the presence of C₂-symmetric bis(oxazoline) ligands. In the best case, a trans/cis ratio of 91:9, with 92% ee for the major isomer, was obtained from the reaction of the p-methoxybenzoate derivative. The highest ee was 95%, for the trans isomer of a 80:20 diastereomer mixture (acetate derivative).

US-A-5 821 374 discloses a process for oxidizing primary and secondary alcohols to the corresponding aldehydes and ketones. The oxidation is carried out by reacting the primary or secondary alcohol with an organic N-chloro compound oxidizing agent in the presence of a catalyst of the formula: 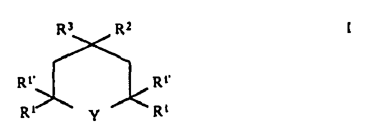 wherein the substituent groups are as defined in the specification of US-A-5 821 374.

### Disclosure of the Invention

According to the method of the present invention, a formylcyclopropanecarboxylate compound of formula (2) can be produced by oxidizing a cyclopropanecarboxylate compound of formula (2) with the advantage of avoiding the problem of waste-treatment or the temperature control and byproduction of the malodorous substance.

That is, the present invention provides a production method of formylcyclopropanecarboxylate compound of formula (2): 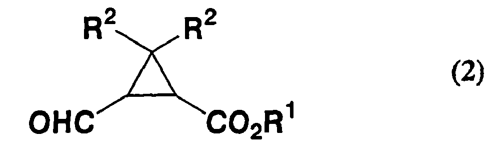 wherein R¹ and R² are as defined below,
which comprises reacting
a cyclopropanecarboxylate compound of formula (1): 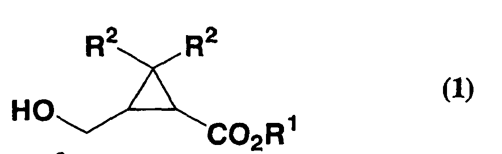 wherein R¹ represent a linear, branched or cyclic alkyl group, a substituted or unsubstituted aryl group, or a substituted or unsubstituted aralkyl group,
R² represents a hydrogen atom or a methyl group, with a hypochlorite in the presence of a nitroxy radical compound.

### Best Mode for Carrying Out the Invention

In the cyclopropanecarboxylate compound of formula (1), hereinafter referred to as the cyclopropanecarboxylate compound (1), examples of the linear, branched or cyclic alkyl group represented by R¹ include, for example, a C1-15 linear, branched or cyclic alkyl group such as methyl, ethyl, n-propyl, isopropyl, n-butyl, isobutyl, sec-butyl, tert-butyl, n-pentyl, n-hexyl, cyclohexyl, n-heptyl, n-octyl, n-nonyl, n-decyl, menthyl, undecyl, dodecyl, tridecyl, tetradecyl, pentadecyl, or the like.

Examples of the substituted or unsubstituted aryl group represented by R¹ include, for example, an unsubstituted aryl group such as phenyl, naphthyl or the like, and such a group as composed of these aryl groups in which one or two or more hydrogen atoms on the aromatic ring are replaced, for example, by a group selected from the alkyl group described above,
a halogen atom such as fluorine, chlorine, or the like,
an alkoxy group such as methoxy, ethoxy or the like,
an aryl group such as phenyl,
an aryloxy group such as phenoxy or the like, or
an alkoxycarbonyl group such as methoxycarbonyl or the like. Specific examples thereof include, for example, 4-methylphenyl group, 2,6-di(tert-butyl)-4-methylphenyl group, a 4-phenoxyphenyl group and the like.

Examples of the substituted or unsubstituted aralkyl group represented by R¹ include, for example, those groups composed of the alkyl group described above and the substituted or unsubstituted aryl group described above, and specific examples thereof include, for example, benzyl, 4-methoxybenzyl, 3-phenoxybenzyl, 2,3,5,6-tetrafluorobenzyl, 2,3,5,6-tetrafluoro-4-methylbenzyl, 2,3,5,6-tetrafluoro-4-methoxymethylbenzyl and the like.

Examples of the cyclopropanecarboxylate compound (1) include, for example,
methyl 2-(hydroxymethyl)cyclopropanecarboxylate,
ethyl 2-(hydroxymethyl)cyclopropanecarboxylate,
n-propyl 2-(hydroxymethyl)cyclopropanecarboxylate,
isopropyl 2-(hydroxymethyl)cyclopropanecarboxylate,
n-butyl 2-(hydroxymethyl)cyclopropanecarboxylate,
isobutyl 2-(hydroxymethyl)cyclopropanecarboxylate,
sec-butyl 2-(hydroxymethyl)cyclopropanecarboxylate,
tert-butyl 2-(hydroxymethyl)cyclopropanecarboxylate,

n-pentyl 2-(hydroxymethyl)cyclopropanecarboxylate,
n-hexyl 2-(hydroxymethyl)cyclopropanecarboxylate,
cyclohexyl 2-(hydroxymethyl)cyclopropanecarboxylate,
n-heptyl 2-(hydroxymethyl)cyclopropanecarboxylate,
n-octyl 2-(hydroxymethyl)cyclopropanecarboxylate,
n-decyl 2-(hydroxymethyl)cyclopropanecarboxylate,
menthyl 2-(hydroxymethyl)cyclopropanecarboxylate,
2,6-di(tert-butyl)-4-methylphenyl 2-(hydroxymethyl)-cyclopropanecarboxylate,
benzyl 2-(hydroxymethyl)cyclopropanecarboxylate,
4-methoxybenzyl 2-(hydroxymethyl)cyclopropanecarboxylate,
3-phenoxybenzyl 2-(hydroxymethyl)cyclopropanecarboxylate,
2,3,5,6-tetrafluoro-4-methoxymethylbenzyl 2-(hydroxylmethyl)cyclopropanecarboxylate,
methyl 3-(hydroxymethyl)-2,2-dimethylcyclopropanecarboxylate,
ethyl 3-(hydroxymethyl)-2,2-dimethylcyclopropanecarboxylate,
n-propyl 3-(hydroxymethyl)-2,2-dimethylcyclopropanecarboxylate,
isopropyl 3-(hydroxymethyl)-2,2-dimethylcyclopropanecarboxylate,
n-butyl 3-(hydroxymethyl)-2,2-dimethylcyclopropanecarboxylate,
isobutyl 3-(hydroxymethyl)-2,2-dimethylcyclopropanecarboxylate,
sec-butyl 3-(hydroxymethyl)-2,2-dimethylcyclopropanecarboxylate,
tert-butyl 3-(hydroxymethyl)-2,2-dimethylcyclopropanecarboxylate,
n-pentyl 3-(hydroxymethyl)-2,2-dimethylcyclopropanecarboxylate,
n-hexyl 3-(hydroxymethyl)-2,2-dimethylcyclopropanecarboxylate,
cyclohexyl 3-(hydroxymethyl)-2,2-dimethylcyclopropanecarboxylate,
n-heptyl 3-(hydroxymethyl)-2,2-dimethylcyclopropanecarboxylate,
n-octyl 3-(hydroxymethyl)-2,2-dimethylcyclopropanecarboxylate,
n-decyl 3-(hydroxymethyl)-2,2-dimethylcyclopropanecarboxylate,
menthyl 3-(hydroxymethyl)-2,2-dimethylcyclopropanecarboxylate,
2,6-di(tert-butyl)-4-methylphenyl 3-(hydroxymethyl)-2,2-dimethylcyclopropanecarboxylate,
benzyl 3-(hydroxymethyl)-2,2-dimethylcyclopropanecarboxylate,
4-methoxybenzyl 3-(hydroxymethyl)-2,2-dimethylcyclopropanecarboxylate,
3-phenoxybenzyl 3-(hydroxymethyl)-2,2-dimethylcyclopropanecarboxylate,
2,3,5,6-tetrafluoro-4-methoxymethylbenzyl 3-(hydroxylmethyl)-2,2-dimethylcyclopropanecarboxylate, and the like.

These cyclopropanecarboxylate compound (1) have two isomers, which are a cis-isomer having the group shown by -CO₂R¹ and the group shown by -CH₂OH on the same side with respect to the cyclopropane ring plane and a trans-isomer having the groups on the opposite side, and any one of the cis-isomer and the trans-isomer or a mixture thereof may be used in the present invention. In addition, the cyclopropanecarboxylate compound (1) has four optical isomers due to the presence of the two asymmetric carbon atoms and any one optical isomer thereof or a mixture of two or more of them may be used.

As the nitroxyl radical compound, a compound having the following structure represented by: 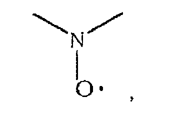 and having no hydrogen atom at the α-position to the nitrogen atom in the structure above may be used, and preferred is a nitroxy radical compound of formula (3): 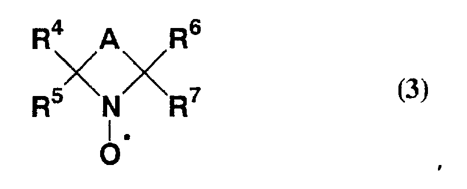 wherein R⁴, R⁵, R⁶ and R⁷ are the same or different and represent
a linear, branched or cyclic lower alkyl group, or
a linear, or branched lower alkenyl group,
an aryl group, an aralkyl group, or an acyl group, and
A represents a group represented by
-CH₂COCH₂-, -COCH₂(CH₂)ₙ-, or -CHXCHY(CHZ)ₙ-, wherein n represents 0 or 1,
X, Y and Z are the same or different and represent a hydrogen atom, a hydroxyl group, a halogen atom, an amino group, an acylamino group, a carbamoyl group,
a linear, branched or cyclic lower alkoxy group,
a lower alkenyloxy group, an aryloxy group,
an aralkyloxy group, or an acyloxy group.

Examples of the linear, branched or cyclic lower alkyl group include, for example, a C1-6 linear, branched or cyclic alkyl group such as methyl, ethyl, n-propyl, isopropyl, n-butyl, isobutyl, sec-butyl, tert-butyl, n-pentyl, neopentyl, cyclopentyl, n-hexyl, cyclohexyl or the like.

Examples of the lower alkenyl group include, for example, a C2-6 linear, or branched alkenyl group such as vinyl , allyl, isopropenyl, 4-pentenyl, 5-hexenyl or the like.

Examples of the aryl group include, for example, a C6-10, aryl group such as phenyl, naphthyl or the like.

Examples of the aralkyl group include, for example, a C1-3 alkyl group substituted with the C6-C10 aryl group, and specific examples thereof include, for example, benzyl, phenylethyl, diphenylmethyl, phenylpropyl and the like.

Examples of the acyl group include, for example, a C1-6 aliphatic acyl group such as formyl, acetyl,propionyl, butyryl, valeryl, pivaloyl, hexanoyl or the like, or a C7-11 aromatic acyl group such as benzoyl or naphthoyl group or the like.

Examples of the halogen atom include, for example, a fluorine atom, a chlorine atom, a bromine atom, an iodine atom, and the like.

Examples of the acylamino group include, for example, an amino group substituted with the aliphatic or aromatic acyl group as described above such as acetylamino group, benzoylamino group or the like.

Examples of the linear, branched or cyclic alkoxy group include, for example, those composed of the lower alkyl groups as described above and an oxygen atom such as a C1-6 linear, branched or cyclic alkoxy group (e.g. methoxy, ethoxy, n-proxy, isoproxy, n-butoxy, sec-butoxy, tert-butoxy, n-pentyloxy, n-hexyloxy, cyclohexyloxy and the like).

Examples of the lower alkenyloxy group include, for example, those groups composed of the linear or branched lower alkenyl group described above and an oxygen atom such as a C2-6 linear or branched alkenyl group, (e.g. vinyloxy, 2-propenyloxy, 1-methylethenyloxy, 4-pentenyloxy, and 5-hexenyloxy and the like).

Examples of the aryloxy group include, for example, those composed of the aryl group as described above and an oxygen atom such as phenoxy, naphthoxy or the like.

Examples of the aralkyloxy group include, for example, those composed of the aralkyl group as described above and an oxygen atom such as a benzyloxy group, a phenethyloxy group or the like.

Examples of the acyloxy group include, for example, those composed of the acyl groups described above and an oxygen atom such as an acetoxy, propionyloxy, benzoyloxy or naphthoyloxy group or the like.

Examples of the nitroxy radical compound include, for example, 2,2,6,6-tetramethylpiperidine-1-oxyl,
4-acetoxy-2,2,6,6-tetramethylpiperidine-1-oxyl,
4-propionyloxy-2,2,6,6-tetramethylpiperidine-1-oxyl,
4-benzoyloxy-2,2,6,6-tetramethylpiperidine-1-oxyl,
4-methoxy-2,2,6,6-tetramethylpiperidine-1-oxyl,
4-ethoxy -2,2,6,6-tetramethylpiperidine-1-oxyl,
4-benzyloxy-2,2,6,6-tetramethylpiperidine-1-oxyl,
4-acetamide-2,2,6,6-tetramethylpiperidine-1-oxyl,
4-oxo-2,2,6,6-tetramethylpiperidine-1-oxyl,
2,2,5,5-tetramethylpyrrolidine-1-oxyl and the like.

As the nitroxy radical compound a commercially available compound or a compound produced according to a similar manner disclosed in JP2002-145861A and the like may be used.

The nitroxy radical compound may be used as it is or after being dissolved or suspend in the solvent described below.

The amount of the nitroxy radical compound that may be used is not limited, and for example, an equivalent amount thereof is sufficient, and preferably, it may be a catalytic amount, which is less than the equivalent amount such as about 0.01 to 10 mol% to the cyclopropanecarboxylate compound (1).

As the oxidizer a hypohalite hereinafter referred to as the oxidizer, is used for the present invention.

Examples of the hypohalite include, for example, an alkali metal or alkaline earth metal salt of hypohalogenic acid such sodium hypochlorite, potassium hypochlorite, calcium hypochlorite, and sodium hypobromite.

The aqueous solution of the alkali metal hypohalite is preferred, more preferred is sodium hypochlorite.

The oxidizer may be used independently, or it may be used in combination as a mixture, moreover, it may be used as it is or it may be used, for example, in a form of an aqueous solution.

The oxidizer is usually used in the amount of about 1 to 5 moles, preferably about 1.5 to 4 moles per 1 mol of the cyclopropanecarboxylate compound (1).

The reaction of the invention is usually carried out by contacting or mixing the nitroxy radical, cyclopropanecarboxylate compound (1), and the oxidizer, and the mixing order is not particularly limited.

The reaction of the present invention is preferably conducted by keeping the pH of the reaction system at a range of from 6 to 13, more preferably at a range of from 6 to 10, still more preferably at a range of from 8 to 10. In order to control the pH of the reaction system, an acid such as a mineral acid or an organic acid, a base such as carbonate, bicarbonate, an alkali metal or alkaline earth metal hydroxide, or an alkali metal or alkaline earth metal carbonate, phosphate, hydrogenphosphate, an optional mixture of the acid, base, phosphate and hydrogenphosphate described above or a suitable buffer solution for maintaining the pH range described above is used.

Examples of the mineral acid include, for example, hydrochloric acid, sulfuric acid, phosphoric acid, boric acid, and the like.

Examples of the organic acid include, for example, acetic acid, propionic acid, benzoic acid, citric acid, p-toluenesulfonic acid, etc.

Examples of the carbonate include, for example, alkali metal carbonate such as sodium carbonate, potassium carbonate, or the like.

Examples of the bicarbonate include, for example, alkali metal hydrogencarbonate such as sodium hydrogencarbonate, potassium hydrogencarbonate or the like.

Examples of the alkali metal hydroxide or the alkaline earth metal hydroxide include, for example, sodium hydroxide, potassium hydroxide, calcium hydroxide, and the like.

Examples of the alkaline earth metal carbonate include, for example, calcium carbonate and the like.

Examples of the phosphate include, for example, alkali metal phosphate such as sodium phosphate, potassium phosphate or the like, and alkali hydrogenphosphate such as potassium dihydrogenphosphate, dipotassium hydrogenphosphate, sodiumdihydrogenphosphate, disodium hydrogenphosphate or the like.

The acid, base, phosphate, hydrogenphosphate or mixtures thereof may be used as it is or may be used, for example, in a form of an aqueous solution. Preferably used is the bicarbonate, and more preferred is, for example, sodium hydrogencarbonate, also preferred is the hydrogenphosphate, and more preferred is, for example, alkali dihydrogenphosphate such as potassium dihydrogenphosphate, sodium dihydrogenphosphate or the like.

An effective amount of the acid, base, phosphate, hydrogenphosphate or mixtures thereof is used to maintain the pH of the aqueous phase of the reaction system within the prescribed pH range, and can be added by selecting a suitable addition method such as an consecutive addition to the reaction system or addition as one portion at one time, and the reaction may be conducted using the same amount irrespective of the use of water solvent or the aqueous solution of them.

In the reaction of the present invention, about 0.1 to 10 moles of bicarbonate or dihydrogenphosphate are usually used per 1 mol of the compound of formula (1).

The reaction of the invention may be conducted in the absence of a solvent, for example, when the cyclopropanecarboxylate compound (1) is liquid, but it is usually conducted in the presence of a solvent. There is no limitation as to the solvent as long as it is inert to the reaction.

Examples of the solvent include, for example, aromatic hydrocarbon solvent such as toluene, xylene or mesitylene, halogenated hydrocarbon solvent such as dichloromethane, chloroform, carbontetrachloride, 1,2-dichloroethane or the like, an ether solvent such as diethyl ether, diisopropyl ether, or methyl tert-butyl ether or the like, a ketone solvent such as methyl isobutyl ketone, methyl tert-butyl ketone, or the like, water and mixtures of the solvents.

The amount of the solvent is not restricted. Moreover, when the reaction is conducted using a water-immiscible organic solvent and water, in a biphase a phase transfer catalyst may be also used in the reaction system.

Examples of the phase transfer catalyst include, for example, a quaternary ammonium halide such as tetra(n-butyl)ammonium bromide, tetra(n-butyl)ammonium chloride, benzyltriethylammonium chloride, or tetraethylammonium chloride or the like.

The reaction temperature is usually -5ºC to 50ºC. After completion of the reaction, an organic phase containing the formylcyclopropanecarboxylate compound of formula (2), hereinafter referred to as formylcyclopropanecarboxylate compound (2), can be obtained, for example, by decomposing remaining oxidizer in the reaction solution with a reducing agent such as sodium thiosulfate, if necessary, and then adding water and/or a water-immiscible organic solvent to extract, and the organic phase is concentrated to isolate the formylcyclopropanecarboxylate compound (2). The isolated formylcyclopropanecarboxylate compound (2) may be further purified, if necessary, by conventionally employed purification process such as distillation, column chromatography or the like.

When an optically active compound is used as the cyclopropanecarboxylate compound (1), optically active formylcyclopropanecarboxylate compound (2) is obtained with the retention of configuration.

Examples of the thus obtained formylcyclopropanecarboxylate compound (2) include, for example,
methyl 2-formylcyclopropanecarboxylate,
ethyl 2-formylcyclopropanecarboxylate,
n-propyl 2-formylcyclopropanecarboxylate,
isopropyl 2-formylcyclopropanecarboxylate,
n-butyl 2-formylcyclopropanecarboxylate,
isobutyl 2-formylcyclopropanecarboxylate,
sec-butyl 2-formylcyclopropanecarboxylate,
tert-butyl 2-formylcyclopropanecarboxylate,
n-pentyl 2-formylcyclopropanecarboxylate,
n-hexyl 2-formylcyclopropanecarboxylate,
cyclohexyl 2-formylcyclopropanecarboxylate,
n-heptyl 2-formylcyclopropanecarboxylate,
n-octyl 2-formylcyclopropanecarboxylate,
n-decyl 2-formylcyclopropanecarboxylate,
menthyl 2-formylcyclopropanecarboxylate,
2,6-di(tert-butyl)-4-methylphenyl 2-formylcyclopropanecarboxylate,
benzyl 2-formylcyclopropanecarboxylate,
4-methoxybenzyl 2-formylcyclopropanecarboxylate,
3-phenoxybenzyl 2-formylcyclopropanecarboxylate,
2,3,5,6-tetrafluoro-4-methoxymethylbenzyl 2-formylcyclopropanecarboxylate,
methyl 2,2-dimethyl-3-formylcyclopropanecarboxylate,
ethyl 2,2-dimethyl-3-formylcyclopropanecarboxylate,
n-propyl 2,2-dimethyl-3-formylcyclopropanecarboxylate,
isopropyl 2,2-dimethyl-3-formylcyclopropanecarboxylate,
n-butyl 2,2-dimethyl-3-formylcyclopropanecarboxylate,
isobutyl 2,2-dimethyl-3-formylcyclopropanecarboxylate,
sec-butyl 2,2-dimethyl-3-formylcyclopropanecarboxylate,
tert-butyl 2,2-dimethyl-3-formylcyclopropanecarboxylate,
n-pentyl 2,2-dimethyl-3-formylcyclopropanecarboxylate,
n-hexyl 2,2-dimethyl-3-formylcyclopropanecarboxylate,
cyclohexyl 2,2-dimethyl-3-formylcyclopropanecarboxylate,

n-heptyl 2,2-dimethyl-3-formylcyclopropanecarboxylate,
n-octyl 2,2-dimethyl-3-formylcyclopropanecarboxylate,
n-decyl 2,2-dimethyl-3-formylcyclopropanecarboxylate, menthyl 2,2-dimethyl-3-formylcyclopropanecarboxylate,
2,6-di(tert-butyl)-4-methylpheny 2,2-dimethyl-3-formylcyclopropanecarboxylate,
benzyl 2,2-dimethyl-3-formylcyclopropanecarboxylate, 4-methoxybenzyl 2,2-dimethyl-3-formylcyclopropanecarboxylate,
3-phenoxybenzyl 2,2-dimethyl-3-formylcyclopropanecarboxylate,
2,3,5,6-tetrafluoro-4-methoxymethylbenzyl 2,2-dimethyl-3-formylcyclopropanecarboxylate and the like.

### Example

The present invention is illustrated below in more detail with Examples, but it is not limited thereto. The analysis was conducted with gas chromatography, Internal standardization method

### Example 1

To a 50 ml Schlenk tube were added 172 mg of ethyl 2,2-dimethyl-3-(hydroxymethyl)cyclopropanecarboxylate, 9.2 g of toluene, 0.18 g of water, 20 mg of dipotassium hydrogenphosphate, and 1.6 mg of 2,2,6,6-tetramethylpiperidine-1-oxyl, and then 931 mg of 12wt% aqueous sodium hypochlorite was added dropwise thereto under stirring at 20 ºC over 2 hrs. After completion of the addition, the reaction solution was maintained under stirring at that temperature for 30 minutes and then 1 ml of aqueous 5 wt% sodium thiosulfate was added thereto and stirred for 5 minutes. After settled, phase separation gave an organic phase containing ethyl 2,2-dimethyl-3-formylcyclopropanecarboxylate.
Yield: 22%.

### Example 2

To a 50 ml Schlenk tube were added 172 mg of ethyl 2,2-dimethyl-3-(hydroxymethyl)cyclopropanecarboxylate, 9.2g of toluene, 0.18 g of water, 20 mg of potassium dihydrogenphosphate, and 1.6 mg of 2,2,6,6-tetramethylpiperidine-1-oxyl, and then the temperature of the mixture was cooled to 0ºC under stirring. 931 mg of 12 wt% aqueous sodium hypochlorite was added dropwise thereto under stirring over 2 hrs. After completion of the addition, the reaction solution was maintained under stirring at that temperature for 30 minutes and then 1 ml of aqueous 5wt% sodium thiosulfate was added thereto and stirred for 5 minutes. After settled, phase separation gave an organic phase containing ethyl 2,2-dimethyl-3-formylcyclopropanecarboxylate.
Yield: 90%.

### Example 3

The organic phase containing ethyl 2,2-dimethyl-3-formylcyclopropanecarboxylate was obtained in a similar manner as in Example 2 except that 13 mg of sodium hydrogencarbonate was employed in place of 20 mg of potassium dihydrogenphosphate employed in Example 2.
Yield: 89%.

### Example 4

To a 50 ml Schlenk tube were added 791 mg of methyl 2,2-dimethyl-3-(hydroxymethyl)cyclopropanecarboxylate, 2.4 g of toluene, 0.80 g of water, 126 mg of sodium hydrogencarbonate, and 7.81 mg of 2,2,6,6-tetramethylpiperidine-1-oxyl, and 9.31 g of 12 wt% aqueous sodium hypochlorite was added dropwise thereto under stirring at 20 QC over 1 hr. The pH was changed from 8.5 to 9.5. After completion of the addition, the reaction solution was maintained under stirring at that temperature for 30 minutes and then 5 ml of aqueous 5 wt% sodium thiosulfate was added thereto and stirred for 5 minutes. After settled, phase separation gave an organic phase containing methyl 2,2-dimethyl-3-formylcyclopropanecarboxylate.
Yield: 97%.

### Example 5

The organic phase containing methyl 2,2-dimethyl-3-formylcyclopropanecarboxylate was obtained in a similar manner as in Example 4 except that 8.94 g of 12 wt% aqueous calcium hypochlorite was employed in place of 9.31 g of 12 wt% aqueous sodium hypochlorite employed in Example 4.
Yield: 29%.

### Example 6

To a 50 ml Schlenk tube were added 791 mg of methyl 2,2-dimethyl-3-(hydroxymethyl)cyclopropanecarboxylate, 24.2 g of toluene, 30.0 g of water, 15.4 mg of citric acid, 829 mg of disodium hydrogenphosphate and 7.81 mg of 2,2,6,6-tetramethylpiperidine-1-oxyl, and 9.31 g of 12 wt% aqueous sodium hypochlorite was added dropwise thereto under stirring at 20º over 1 hr. The pH of the aqueous phase changed from 8 to 10 during the reaction. After completion of the addition, the reaction solution was maintained under stirring at that temperature for 30 minutes and then 5 ml of aqueous 5wt% sodium thiosulfate was added thereto and stirred for 5 minutes. After settled, phase separation gave an organic phase containing methyl 2,2-dimethyl-3-formylcyclopropanecarboxylate.
Yield: 64%.

### Example 7

The organic phase containing methyl 2,2-dimethyl-3-formylcyclopropanecarboxylate was obtained in a similar manner as in Example 6 except that 217 mg of citric acid and 534 mg of disodium dygrogenphosphate were used in place of 15.4 mg of citric acid and 829 mg of disodium hygrogenphosphate. The pH of the aqueous phase of the reaction solution changed from 6 to 9 during the reaction.
Yield: 19%.

### Example 8

The organic phase containing methyl 2,2-dimethyl-3-formylcyclopropanecarboxylate was obtained in a similar manner as in Example 6 except that 45.9 mg of sodium hydrogencarbonate and 43.7 mg of sodium hydroxide were used in place of 15.4 mg of citric acid and 829 mg of disodium hygrogenphosphate. The pH of aqueous phase of reaction solution changed from 10 to 11.
Yield: 12%.

### Industrial Applicability

According to the method of the present invention, formylcyclopropanecarboxylate compound, which is a useful as synthetic intermediate for pyrethroid or furanone derivative can be produced with the advantage of avoiding a troublesome after-treatment after the reaction and byproduction of a malodorous substance (e.g. JP46-24695B, U.S. H No. 49, Tetrahedron, 57, 6083 (2001)).

## Claims

1. A production method of formylcyclopropanecarboxylate compound of formula (2): 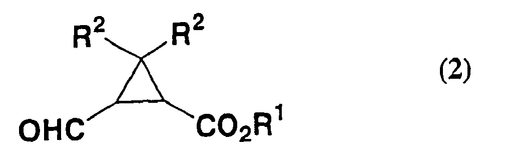 wherein R¹ and R² are as defined below,
which comprises reacting
a cyclopropanecarboxylate compound of formula (1): 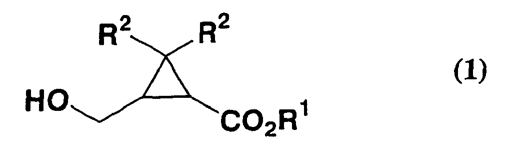 wherein R¹ represents a linear, branched or cyclic alkyl group, a substituted or unsubstituted aryl group, or a substituted or unsubstituted aralkyl group,
R² represents a hydrogen atom or a methyl group, with a hypochlorite in the presence of a nitroxy radical compound.

2. A production method according to claim 1, wherein the nitroxy radical compound is a nitroxy radical compound of formula (3): 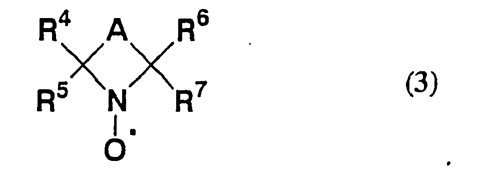 wherein R⁴, R⁵, R⁶ and R⁷ are the same or different and represent
a linear, branched or cyclic lower alkyl group, or
a linear or branched lower alkenyl group,
an aryl group, an aralkyl group, or an acyl group, and
A represents the group represented by
- CH₂COCH₂-, -COCH₂(CH₂)ₙ-, or -CHXCHY(CHZ)ₙ-,
wherein n represents 0 or 1,
X, Y and Z are the same or different and represent a hydrogen atom, a hydroxyl group, a halogen atom, an amino group, an acylamino group, a carbamoyl group, a linear, branched or cyclic lower alkoxy group, a lower alkenyloxy group, an aryloxy group, an aralkyloxy group, or an acyloxy group.

3. A production method according to claim 2, wherein nitroxy radical compound of formula (3) is 2,2,6,6-tetramethylpiperidine-1-oxyl.

4. A production method according to claim 1 or 2, wherein the reaction is conducted at a pH range of 6-13.

5. A production method according to claim 4, wherein the reaction is conducted at a pH range of 8-10.

6. A production method according to claim 4, wherein the reaction is conducted in the presence of hydrogencarbonate or hydrogenphosphate.

7. A production method according to claim 5, wherein the reaction is conducted in the presence of hydrogencarbonate or hydrogenphosphate.

## Patentansprüche

1. Verfahren zur Herstellung einer Formylcyclopropancarboxylatverbindung der Formel (2): 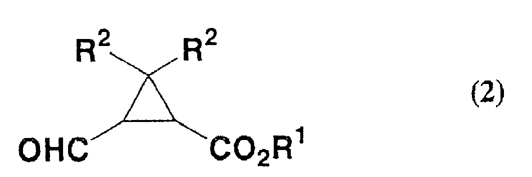 worin R¹ und R² wie im Folgenden angegeben definiert sind, wobei das Verfahren die Umsetzung einer Cyclopropancarboxylatverbindung der Formel (1): 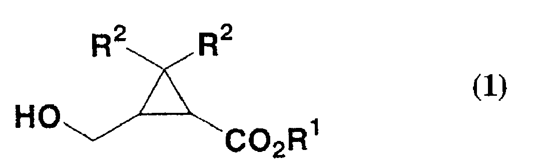 worin R¹ für eine lineare, verzweigte oder cyclische Alkylgruppe, eine substituierte oder unsubstituierte Arylgruppe oder eine substituierte oder unsubstituierte Aralkylgruppe steht,
R² für ein Wasserstoffatom oder eine Methylgruppe steht, mit einem Hypochlorit in Gegenwart einer Nitroxyradikalverbindung umfasst.

2. Herstellungsverfahren nach Anspruch 1, wobei die Nitroxyradikalverbindung eine Nitroxyradikalverbindung der Formel (3) ist: 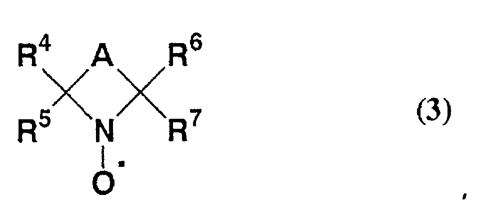 worin
R⁴, R⁵, R⁶ und R⁷ gleich oder verschieden sind und für eine lineare, verzweigte oder cyclische Niederalkylgruppe oder eine lineare oder verzweigte Niederalkenylgruppe, eine Arylgruppe, eine Aralkylgruppe oder eine Acylgruppe stehen und
A für eine Gruppe von -CH₂COCH₂-, -COCH₂(CH₂)ₙ- oder -CHXCHY(CHZ)ₙ- steht,
worin
n für 0 oder 1 steht,
X, Y und Z gleich oder verschieden sind und für ein Wasserstoffatom, eine Hydroxylgruppe, ein Halogenatom, eine Aminogruppe, eine Acylaminogruppe, eine Carbamoylgruppe, eine lineare, verzweigte oder cyclische Niederalkoxygruppe, eine Niederalkenyloxygruppe, eine Aryloxygruppe, eine Aralkyloxygruppe oder eine Acyloxygruppe stehen.

3. Herstellungsverfahren nach Anspruch 2, wobei die Nitroxyradikalverbindung der Formel (3) 2,2,6,6-Tetramethylpiperidin-1-oxyl ist.

4. Herstellungsverfahren nach Anspruch 1 oder 2, wobei die Umsetzung in einem pH-Bereich von 6-13 durchgeführt wird.

5. Herstellungsverfahren nach Anspruch 4, wobei die Umsetzung in einem pH-Bereich von 8-10 durchgeführt wird.

6. Herstellungsverfahren nach Anspruch 4, wobei die Umsetzung in Gegenwart von Hydrogencarbonat oder Hydrogenphosphat durchgeführt wird.

7. Herstellungsverfahren nach Anspruch 5, wobei die Umsetzung in Gegenwart von Hydrogencarbonat oder Hydrogenphosphat durchgeführt wird.

## Revendications

1. Procédé de production de cyclopropanecarboxylate de formyle de formule (2) : 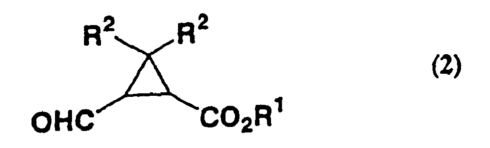 dans laquelle R¹ et R² sont tels que définis ci-dessous, qui comprend la mise en réaction
d'un composé de cyclopropanecarboxylate de formule (1) : 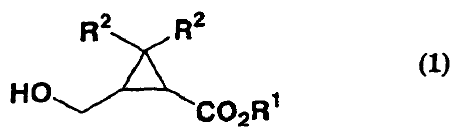 dans laquelle R¹ représente un groupe alkyle linéaire, ramifié ou cyclique, un groupe aryle substitué ou non substitué, ou un groupe aralkyle substitué ou non substitué,
R² représente un atome d'hydrogène ou un groupe méthyle, avec un hypochlorite en présence d'un composé comprenant un radical nitroxy.

2. Procédé de production selon la revendication 1, dans lequel le composé comprenant un radical nitroxy est un composé comprenant un radical nitroxy de formule (3) : 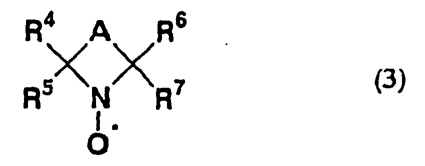 dans laquelle R⁴, R⁵, R⁶ et R⁷ sont identiques ou différents et représentent
un groupe alkyle inférieur linéaire, ramifié ou cyclique, ou
un groupe alcényle inférieur linéaire ou ramifié,
un groupe aryle, un groupe aralkyle ou un groupe acyle, et
A représente le groupe représenté par -CH₂COCH₂-, -COCH₂(CH₂)ₙ- ou -CHXCHY(CHZ)ₙ-,
où n représente 0 ou 1,
X, Y et z sont identiques ou différents et représentent un atome d'hydrogène, un groupe hydroxyle, un atome d'halogène, un groupe amino, un groupe acylamino, un groupe carbamoyle, un groupe alcoxy inférieur linéaire, ramifié ou cyclique, un groupe alcényloxy inférieur, un groupe aryloxy, un groupe aralkyloxy, ou un groupe acyloxy.

3. Procédé de production selon la revendication 2, dans lequel le composé comprenant un radical nitroxy de formule (3) est le 2,2,6,6-tétraméthylpipéridine-1-oxyle.

4. Procédé de production selon la revendication 1 ou 2, dans lequel la réaction est réalisée dans une plage de pH allant de 6 à 13.

5. Procédé de production selon la revendication 4, dans lequel la réaction est réalisée dans une plage de pH allant de 8 à 10.

6. Procédé de production selon la revendication 4, dans lequel la réaction est réalisée en présence d'hydrogénocarbonate ou d'hydrogénophosphate.

7. Procédé de production selon la revendication 5, dans lequel la réaction est réalisée en présence d'hydrogénocarbonate ou d'hydrogénophosphate.
